(19) Europäisches Patentamt
European Patent Office
Office européen des brevets

(11) Veröffentlichungsnummer: **0 010 166**
**B1**

(12) # EUROPÄISCHE PATENTSCHRIFT

(45) Veröffentlichungstag der Patentschrift: **27.10.82**

(21) Anmeldenummer: **79103485.3**

(22) Anmeldetag: **17.09.79**

(51) Int. Cl.[3]: **C 07 D 231/12**, C 07 D 261/08, C 07 D 275/02, A 01 N 43/56, A 01 N 43/80 // C07C103/365

(54) **N-(1,2-Azolyl)alkyl-chloracetanilide, Verfahren zu ihrer Herstellung sowie ihre Verwendung als Herbizide.**

(30) Priorität: **28.09.78 DE 2842315**

(43) Veröffentlichungstag der Anmeldung: **30.04.80 Patentblatt 80/9**

(45) Bekanntmachung des Hinweises auf die Patenterteilung: **27.10.82 Patentblatt 82/43**

(84) Benannte Vertragsstaaten: **AT BE CH DE FR GB IT NL**

(56) Entgegenhaltungen:
**DE-A-2 704 281**
**DE-A-2 742 583**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Stetter, Jörg, Dr., Pahlkestrasse 3, D-5600 Wuppertal 1 (DE)**
Erfinder: **Ditgens, Klaus, Dr., Claudiusweg 7, D-5600 Wuppertal 1 (DE)**
Erfinder: **Thomas, Rudolf, Dr., Wilkhausstrasse 129, D-5600 Wuppertal 2 (DE)**
Erfinder: **Eue, Ludwig, Dr., Paul-Klee-Strasse 36, D-5090 Leverkusen 1 (DE)**
Erfinder: **Schmidt, Robert Rudolf, Dr., Hahnenweg 5, D-5000 Köln 80 (DE)**

**EP 0 010 166 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

N-(1,2-Azolyl)alkyl-chloracetanilide,
Verfahren zu ihrer Herstellung sowie ihre Verwendung als Herbizide

Die vorliegende Erfindung betrifft neue N-(1,2-Azolyl)-alkyl-chloracetanilide, mehrere Verfahren zu ihrer Herstellung sowie ihre Verwendung als Herbizide.

Es ist bereits bekanntgeworden, daß man 2,6-Diethyl-N-methoxymethyl-chloracetanilid zur selektiven Unkrautbekämpfung verwenden kann (vgl. R. Wegler, Chemie der Pflanzenschutz- und Schädlingsbekämpfungsmittel, Band 5, Seite 225, Springer-Verlag (1977)). Diese Verbindung ist jedoch nicht immer ausreichend wirksam und in ihrer Selektivität nicht immer ganz befriedigend.

Weiterhin ist bereits bekanntgeworden, daß zahlreiche N-substituierte Halogenacetanilide, in denen das Stickstoffatom des Analinteiles über eine $CH_2$-Gruppe mit einem gegebenenfalls substituierten Azol über ein N-Atom des Azol-Restes verbunden ist, gute herbizide Eigenschaften besitzen (vgl. DE-A-2 704 281 und DE-A-2 742 583). Die selektive herbizide Wirksamkeit dieser Stoffe läßt jedoch in manchen Fällen zu wünschen übrig.

Es wurden nun neue N-(1,2-Azolyl)-alkyl-chloracetanilide der Formel

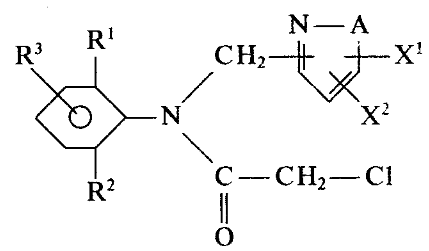

(I)

in welcher

A für Sauerstoff, Schwefel oder die Gruppierung > NR steht, wobei
R für Wasserstoff oder Alkyl mit 1 bis 4 Kohlenstoffatomen steht,
$R^1$ für Alkyl mit 1 bis 4 Kohlenstoffatomen steht,
$R^2$ für Wasserstoff oder Alkyl mit 1 bis 4 Kohlenstoffatomen steht,
$R^3$ für Wasserstoff oder Alkyl mit 1 bis 4 Kohlenstoffatomen steht,
$X^1$ für Wasserstoff oder Alkyl mit 1 bis 4 Kohlenstoffatomen steht und
$X^2$ für Wasserstoff oder Alkyl mit 1 bis 4 Kohlenstoffatomen steht

und der Azolyl-Rest über ein Kohlenstoffatom gebunden ist, gefunden.

Weiterhin wurde gefunden, daß man die N-(1,2-Azolyl)-alkyl-chloracetanilide der Formel (I) erhält, wenn man

a) N-(1,2-Azolyl)alkyl-aniline der Formel

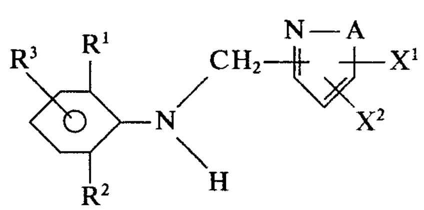

(II)

in welcher
A, $R^1$, $R^2$, $R^3$, $X^1$ und $X^2$ die oben angebene Bedeutung haben, und der Azolyl-Rest über ein Kohlenstoffatom gebunden ist,
mit Chloressigsäurechlorid oder -bromid bzw. -anhydrid der Formeln

$$Cl-CH_2-CO-Cl(Br) \qquad (IIIa)$$

bzw.

$$(Cl-CH_2-CO)_2O \qquad (IIIb)$$

in Gegenwart eines Verdünnungsmittels und gegebenenfalls in Gegenwart eines Säurebindemittels umsetzt, oder

b) Chloracetanilide der Formel

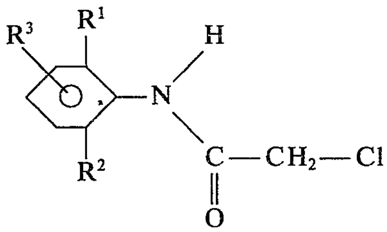

(IV)

,

in welcher
$R^1$, $R^2$ und $R^3$ die oben angebene Bedeutung haben,
mit Azolyl-alkyl-Derivaten der Formel

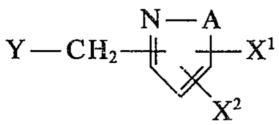

(V)

in welcher
A, $X^1$ und $X^2$ die oben angegebene Bedeutung haben und
Y für Halogen, den Mesylat- oder Tosylat-Rest steht, und der Azolyl-Rest über ein Kohlenstoffatom gebunden ist,
in Gegenwart eines Säurebinders und gegebenenfalls in Gegenwart eines organischen Lösungsmittels umsetzt.

Die neuen N-(1,2-Azolyl)alkyl-chloracetanilide der Formel (I) weisen starke herbizide, insbesondere selektivherbizide Eigenschaften auf.

Überraschenderweise zeigen die erfindungsgemäßen N-(1,2-Azolyl)alkyl-chloracetanilide bei sehr guter Unkrautwirkung bessere Möglichkeiten zum Einsatz als selektive Unkrautbekämpfungsmittel in wichtigen Kulturpflanzen als das vorbekannte 2,6-Diethyl-N-methoxy-methyl-chlor-acetanilid, welches ein gut wirksamer Stoff gleicher Wirkungsart ist. Ferner übertreffen die erfindungsgemäßen N-(1,2-Azolyl)-alkyl-chloracetanilide auch konstitutionell ähnliche Stoffe, die in der DE-A-2 704 281 bzw. in der DE-A-2 742 583 offenbart werden, bezüglich ihrer selektiven herbiziden Wirksamkeit.

Die erfindungsgemäßen N-(1,2-Azolyl)alkyl-chloracetanilide sind durch die Formel (I) allgemein definiert. Besonders bevorzugt sind diejenigen Stoffe der Formel (I), in denen A für Sauerstoff, Schwefel oder die Gruppierung >NR steht, wobei R für Methyl, Ethyl, Propyl oder Butyl steht; $R^1$ für Methyl, Ethyl, Isopropyl, sek.-Butyl oder tert.-Butyl steht; $R^2$ für Wasserstoff, Methyl, Ethyl, Isopropyl, sek.-Butyl oder tert.-Butyl steht; $R^3$ für Wasserstoff, Methyl, Ethyl, Isopropyl, sek.-Butyl oder tert.-Butyl steht, $X^1$ und $X^2$ gleich oder verschieden sind und für Wasserstoff, Methyl, Ethyl, n-Propyl, Isopropyl, n-Butyl, sek.-Butyl oder tert.-Butyl stehen.

Im einzelnen seien außer den bei den Herstellungsbeispielen genannten Verbindungen die folgenden Verbindungen der allgemeinen Formel (I) genannt:

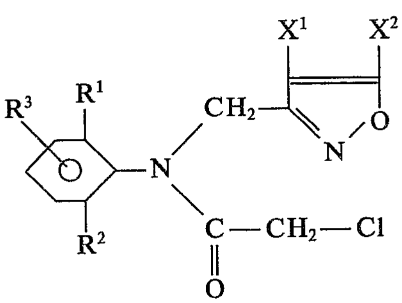

(Ia)

| $R^1$ | $R^2$ | $R^3$ | $X^1$ | $X^2$ |
|---|---|---|---|---|
| $CH_3$ | $CH_3$ | H | H | H |
| $C_2H_5$ | $CH_3$ | H | H | H |
| $C_2H_5$ | $C_2H_5$ | H | H | H |

Fortsetzung

| $R^1$ | $R^2$ | $R^3$ | $X^1$ | $X^2$ |
|---|---|---|---|---|
| $C(CH_3)_3$ | H | H | H | H |
| $CH_3$ | H | 3-$CH_3$ | H | H |
| $CH_3$ | H | 5-$CH_3$ | H | H |
| $CH_3$ | $CH_3$ | 3-$CH_3$ | H | H |
| $CH_3$ | $CH_3$ | H | H | $CH_3$ |
| $C_2H_5$ | $CH_3$ | H | H | $CH_3$ |
| $C_2H_5$ | $C_2H_5$ | H | H | $CH_3$ |
| $C(CH_3)_3$ | H | H | H | $CH_3$ |
| $CH_3$ | H | 3-$CH_3$ | H | $CH_3$ |
| $CH_3$ | H | 5-$CH_3$ | H | $CH_3$ |
| $CH_3$ | $CH_3$ | 3-$CH_3$ | H | $CH_3$ |
| $CH_3$ | $CH_3$ | H | H | $C_2H_5$ |
| $C_2H_5$ | $CH_3$ | H | H | $C_2H_5$ |
| $C_2H_5$ | $C_2H_5$ | H | H | $C_2H_5$ |
| $CH_3$ | $CH_3$ | H | $CH_3$ | H |
| $C_2H_5$ | $CH_3$ | H | $CH_3$ | H |
| $C_2H_5$ | $C_2H_5$ | H | $CH_3$ | H |
| $CH_3$ | $CH_3$ | H | $CH_3$ | $CH_3$ |
| $C_2H_5$ | $CH_3$ | H | $CH_3$ | $CH_3$ |
| $C_2H_5$ | $C_2H_5$ | H | $CH_3$ | $CH_3$ |
| $CH_3$ | $CH_3$ | H | H | $C(CH_3)_3$ |
| $C_2H_5$ | $CH_3$ | H | H | $C(CH_3)_3$ |
| $C_2H_5$ | $C_2H_5$ | H | H | $C(CH_3)_3$ |
| $CH_3$ | $CH_3$ | H | H | $C_3H_7$ |
| $C_2H_5$ | $CH_3$ | H | H | $C_3H_7$ |
| $C_2H_5$ | $C_2H_5$ | H | H | $C_3H_7$ |

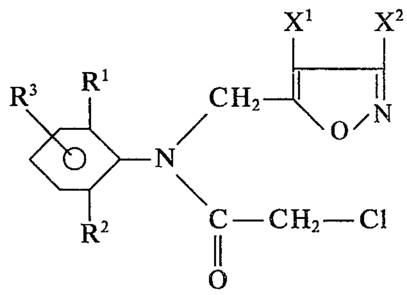

(Ib)

| $R^1$ | $R^2$ | $R^3$ | $X^1$ | $X^2$ |
|---|---|---|---|---|
| $CH_3$ | $C_2H_5$ | H | H | H |
| $C_2H_5$ | $C_2H_5$ | H | H | H |
| $CH_3$ | $C_2H_5$ | H | H | $CH_3$ |
| $C_2H_5$ | $C_2H_5$ | H | H | $CH_3$ |
| $CH_3$ | $C_2H_5$ | H | H | i-$C_3H_7$ |
| $C_2H_5$ | $C_2H_5$ | H | H | i-$C_3H_7$ |
| $CH_3$ | $C_2H_5$ | H | H | $C(CH_3)_3$ |
| $C_2H_5$ | $C_2H_5$ | H | H | $C(CH_3)_3$ |

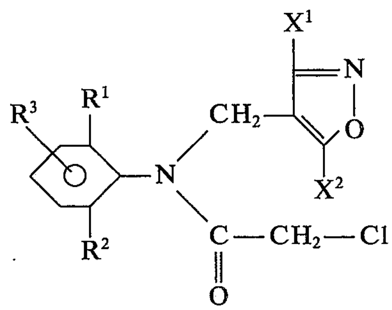

(Ic)

| $R^1$ | $R^2$ | $R^3$ | $X^1$ | $X^2$ |
|---|---|---|---|---|
| $CH_3$ | $C_2H_5$ | H | H | H |
| $C_2H_5$ | $C_2H_5$ | H | H | H |
| $CH_3$ | $C_2H_5$ | H | $CH_3$ | H |
| $C_2H_5$ | $C_2H_5$ | H | $CH_3$ | H |
| $CH_3$ | $C_2H_5$ | H | $CH_3$ | $CH_3$ |
| $C_2H_5$ | $C_2H_5$ | H | $CH_3$ | $CH_3$ |
| $CH_3$ | $CH_3$ | H | $CH_3$ | $CH_3$ |

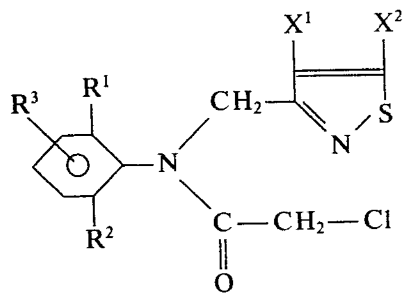

(Id)

| $R^1$ | $R^2$ | $R^3$ | $X^1$ | $X^2$ |
|---|---|---|---|---|
| $CH_3$ | $CH_3$ | H | H | H |
| $C_2H_5$ | $CH_3$ | H | H | H |
| $C_2H_5$ | $C_2H_5$ | H | H | H |
| $C(CH_3)_3$ | H | H | H | H |
| $CH_3$ | H | 3-$CH_3$ | H | H |
| $CH_3$ | H | 5-$CH_3$ | H | H |
| $C_2H_5$ | $C_2H_5$ | H | H | $CH_3$ |
| $CH_3$ | $C_2H_5$ | H | H | $CH_3$ |
| $CH_3$ | $CH_3$ | H | H | $CH_3$ |
| $C_2H_5$ | $C_2H_5$ | H | $CH_3$ | H |
| $CH_3$ | $C_2H_5$ | H | $CH_3$ | H |
| $CH_3$ | $CH_3$ | H | $CH_3$ | H |
| $C(CH_3)_3$ | H | H | $CH_3$ | H |

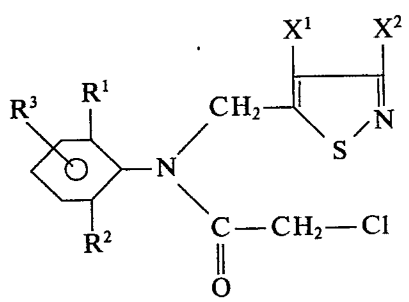

(Ie)

| $R^1$ | $R^2$ | $R^3$ | $X^1$ | $X^2$ |
|---|---|---|---|---|
| $CH_3$ | $CH_3$ | H | H | $CH_3$ |
| $C_2H_5$ | $CH_3$ | H | H | $CH_3$ |
| $C_2H_5$ | $C_2H_5$ | H | H | $CH_3$ |

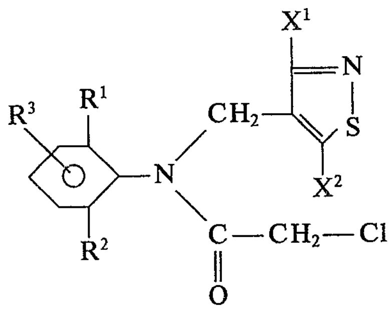

(If)

| $R^1$ | $R^2$ | $R^3$ | $X^1$ | $X^2$ |
|---|---|---|---|---|
| $CH_3$ | $C_2H_5$ | H | $CH_3$ | H |
| $C_2H_5$ | $C_2H_5$ | H | $CH_3$ | H |
| $CH_3$ | $C_2H_5$ | H | $CH_3$ | $CH_3$ |

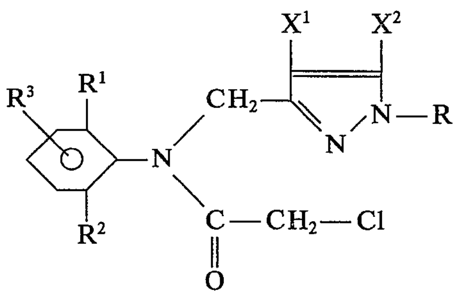

(Ig)

| $R^1$ | $R^2$ | $R^3$ | $X^1$ | $X^2$ | R |
|---|---|---|---|---|---|
| $CH_3$ | $CH_3$ | H | H | H | $CH_3$ |
| $C_2H_5$ | $CH_3$ | H | H | H | $CH_3$ |
| $C_2H_5$ | $C_2H_5$ | H | H | H | $CH_3$ |
| $CH_3$ | H | 3-$CH_3$ | H | H | $CH_3$ |
| $CH_3$ | H | 5-$CH_3$ | H | H | $CH_3$ |
| $CH_3$ | $CH_3$ | H | H | $CH_3$ | $CH_3$ |
| $CH_3$ | $C_2H_5$ | H | H | $CH_3$ | $CH_3$ |
| $C_2H_5$ | $C_2H_5$ | H | H | $CH_3$ | $CH_3$ |
| $C(CH_3)_3$ | H | H | H | $CH_3$ | $CH_3$ |
| $CH_3$ | H | 3-$CH_3$ | H | $CH_3$ | $CH_3$ |
| $CH_3$ | H | 5-$CH_3$ | H | $CH_3$ | $CH_3$ |
| $CH_3$ | $C_2H_5$ | H | H | H | $C_2H_5$ |
| $CH_3$ | $CH_3$ | H | H | H | $C_2H_5$ |
| $C_2H_5$ | $C_2H_5$ | H | H | H | $C_2H_5$ |
| $CH_3$ | $CH_3$ | H | H | H | $C_3H_7$ |

Fortsetzung

| $R^1$ | $R^2$ | $R^3$ | $X^1$ | $X^2$ | R |
|---|---|---|---|---|---|
| $C_2H_5$ | $C_2H_5$ | H | H | H | $C_3H_7$ |
| $CH_3$ | $C_2H_5$ | H | H | H | $C_3H_7$ |
| $CH_3$ | $CH_3$ | H | H | H | $C_4H_9$ |
| $C_2H_5$ | $C_2H_5$ | H | H | H | $C_4H_9$ |
| $C_2H_5$ | $CH_3$ | H | H | H | $C_4H_9$ |
| $CH_3$ | $CH_3$ | H | H | $CH_3$ | $C_2H_5$ |
| $CH_3$ | $C_2H_5$ | H | H | $CH_3$ | $C_2H_5$ |
| $C_2H_5$ | $C_2H_5$ | H | H | $CH_3$ | $C_2H_5$ |
| $CH_3$ | $CH_3$ | H | $CH_3$ | $CH_3$ | $CH_3$ |
| $CH_3$ | $C_2H_5$ | H | $CH_3$ | $CH_3$ | $CH_3$ |
| $CH_3$ | $CH_3$ | H | $CH_3$ | H | $CH_3$ |
| $C_2H_5$ | $C_2H_5$ | H | $CH_3$ | H | $CH_3$ |
| $CH_3$ | $C_2H_5$ | H | $CH_3$ | H | $CH_3$ |

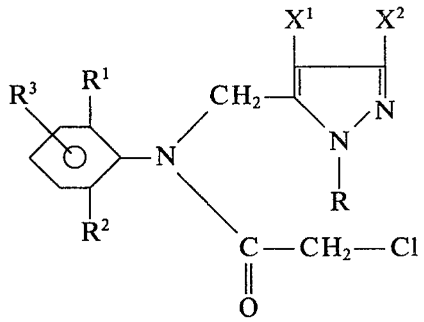

(Ih)

| $R^1$ | $R^2$ | $R^3$ | $X^1$ | $X^2$ | R |
|---|---|---|---|---|---|
| $CH_3$ | $CH_3$ | H | H | $CH_3$ | $CH_3$ |
| $CH_3$ | $C_2H_5$ | H | H | $CH_3$ | $CH_3$ |
| $C_2H_5$ | $C_2H_5$ | H | H | $CH_3$ | $CH_3$ |
| $CH_3$ | $CH_3$ | H | H | H | $CH_3$ |
| $CH_3$ | $C_2H_5$ | H | H | H | $CH_3$ |
| $C_2H_5$ | $C_2H_5$ | H | H | H | $CH_3$ |

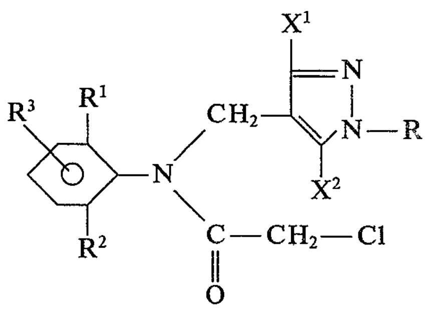

(Ii)

| $R^1$ | $R^2$ | $R^3$ | $X^1$ | $X^2$ | R |
|---|---|---|---|---|---|
| $CH_3$ | $C_2H_5$ | H | $CH_3$ | $CH_3$ | $CH_3$ |
| $C_2H_5$ | $C_2H_5$ | H | $CH_3$ | $CH_3$ | $CH_3$ |
| $CH_3$ | $C_2H_5$ | H | H | H | $CH_3$ |
| $C_2H_5$ | $C_2H_5$ | H | H | H | $CH_3$ |

Verwendet man 2-Ethyl-6-methyl-N-(3′-tert.-butyl-isoxazol-5′-yl-methyl)-anilin und Chloracetylchlorid als Ausgangsstoffe, so kann der Reaktionsablauf durch das folgende Formelschema wiedergegeben werden (Verfahrensvariante a):

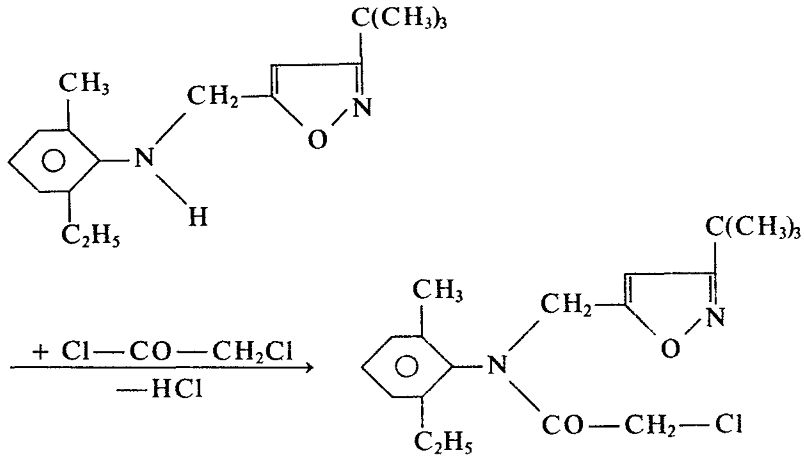


Verwendet man 2-Ethyl-6-methyl-chloracetanilid und 5-Brom-methyl-3-methyl-isoxazol als Ausgangsstoffe, so kann der Reaktionsablauf durch das folgende Formelschema wiedergegeben werden (Verfahrensvariante b):

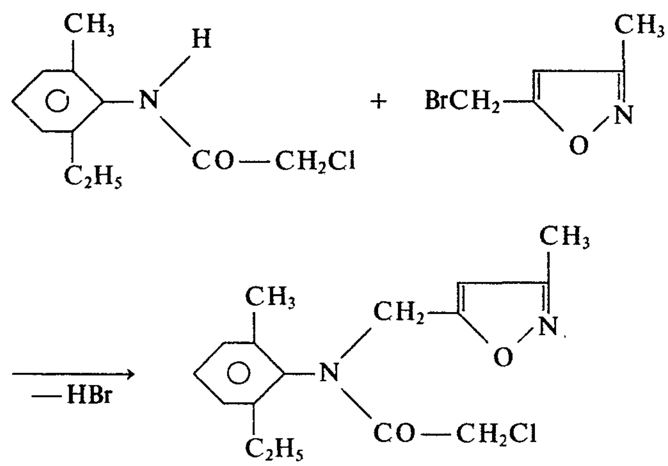


Die bei der Durchführung des erfindungsgemäßen Verfahrens (a) als Ausgangsstoffe benötigten N-Azolylalkylaniline sind durch die Formel (II) allgemein definiert. In dieser Formel stehen A, $R^1$, $R^2$, $R^3$, $X^1$ und $X^2$ für diejenigen Reste, die bereits im Zusammenhang mit der Beschreibung der erfindungsgemäßen Stoffe der Formel (I) für diese Reste genannt wurden.

Die N-(1,2-Azolyl)-alkyl-aniline der Formel (II) sind noch nicht bekannt. Man erhält sie, wenn man Aniline der Formel

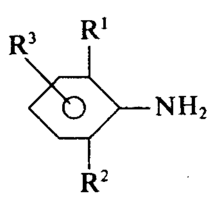

(VI)

in welcher

$R^1$, $R^2$ und $R^3$ die oben angegebene Bedeutung haben,

α) mit Azolylalkyl-Derivaten der Formel

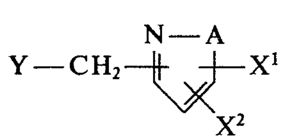

(V)

in welcher

A, $X^1$, $X^2$ und Y die oben angegebene Bedeutung haben und der Azolyl-Rest über ein Kohlenstoffatom gebunden ist,
in Gegenwart eines Säurebinders und gegebenenfalls in Gegenwart eines Verdünnungsmittels umsetzt,
oder

β) mit Azol-aldehyden der Formel

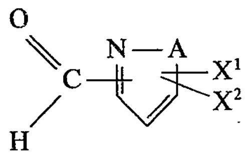

(VII)

in welcher
A, $X^1$ und $X^2$ die oben angegebene Bedeutung haben, und der Azolyl-Rest über ein Kohlenstoffatom gebunden ist,
in Gegenwart eines inerten organischen Lösungsmittels und gegebenenfalls eines Katalysators umsetzt und die entstehenden Imine der Formel

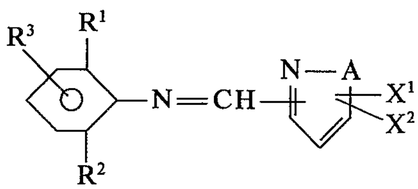

(VIII)

in welcher
A, $R^1$, $R^2$, $R^3$, $X^1$ und $X^2$ die oben angegebene Bedeutung haben und der Azolyl-Rest über ein Kohlenstoffatom gebunden ist,
gegebenenfalls in Gegenwart eines polaren Verdünnungsmittels reduziert.

Die bei der Herstellung der N-(1,2-Azolyl)alkyl-aniline der Formel (II) als Ausgangsstoffe benötigten Aniline der Formel (VI) sind allgemein bekannte Verbindungen der organischen Chemie. Als Beispiele seien genannt:

Anilin, 2-Methylanilin, 2-Ethylanilin, 2-Isopropylanilin, 2-sek.-Butylanilin, 2-tert.-Butylanilin, 2,6-Dimethylanilin, 2,3-Dimethylanilin, 2,5-Dimethylanilin, 3,5-Di-methylanilin, 2,6-Diethylanilin, 2-Ethyl-6-methylanilin, 2,4,6-Trimethylanilin, 2-Ethyl-4,6-dimethylanilin, 2,6-Diethyl-4-methylanilin, 2,6-Diisopropyl-4-methylanilin, 2,3,6-Trimethylanilin.

Die bei der Herstellung der N-(1,2-Azolyl)alkyl-aniline der Formel (II) weiterhin als Ausgangsstoffe benötigten Azol-aldehyde der Formel (VII) sind bekannt (vgl. z. B. Arch. Pharm. 264 (1926); J. Chem. Soc. 1957, 3314 und 1964, 3114) bzw. können nach den dort beschriebenen Verfahren erhalten werden.

Bei der Herstellung der N-(1,2-Azolyl(alkyl-aniline der Formel (II) nach dem Verfahren (α) können als Säurebinder alle üblichen Säureakzeptoren verwendet werden. Vorzugsweise in Betracht kommen Alkalicarbonate wie Kalium- oder Natriumcarbonat.

Als Verdünnungsmittel können bei dem Verfahren (α) alle üblichen inerten organischen Lösungsmittel eingesetzt werden. Vorzugsweise in Betracht kommen Dimethylformamid und Toluol.

Die Reaktionstemperaturen können bei dem Verfahren (α) innerhalb eines größeren Bereiches variiert werden. Im allgemeinen arbeitet man zwischen 0° C und 180° C, vorzugsweise zwischen 20° C und 160° C.

Bei der Umsetzung nach dem Verfahren (α) setzt man die Aniline der Formel (VI) und die Azolylalkyl-Derivate der Formel (V) im allgemeinen in äquimolaren Mengen ein. Es ist jedoch auch möglich, eine der Komponenten, vorzugsweise das Anilin der Formel (VI), in einem Überschuß einzusetzen. Die Aufarbeitung und Isolierung der Reaktionsprodukte erfolgt nach üblichen Methoden.

Bei der Herstellung der N-(1,2-Azolyl)alkylaniline der Formel (II) nach dem Verfahren (β) können in der ersten Stufe als inerte organische Lösungsmittel alle üblichen derartigen Solventien verwendet werden. Vorzugsweise in Betracht kommen aromatische Lösungsmittel, wie Toluol.

Als Katalysatoren können bei der Umsetzung (β) in der ersten Stufe alle für derartige Additionen üblichen Reaktionsbeschleuniger verwendet werden. Vorzugsweise in Frage kommen starke organische Säuren, wie p-Toluolsulfonsäure.

In der zweiten Stufe des Verfahrens (β) können als Lösungsmittel alle inerten polaren organischen Solventien verwendet werden. Vorzugsweise in Betracht kommen Alkohole, wie Methanol.

Als Reduktionsmittel können bei der Durchführung der zweiten Stufe des Verfahrens (β) vorzugsweise komplexe Hydride, wie zum Beispiel Natriumborhydrid, verwendet werden.

Die Reaktionstemperaturen können bei der Durchführung des Verfahrens (β) sowohl in der ersten als auch in der zweiten Stufe innerhalb eines größeren Bereiches variiert werden. In der ersten Stufe

arbeitet man im allgemeinen bei Temperaturen zwischen 40° C und 140° C, vorzugsweise zwischen 60° C und 120° C. In der zweiten Stufe arbeitet man im allgemeinen bei Temperaturen zwischen −10° C und +100° C, vorzugsweise zwischen 0° C und 80° C.

Bei der Durchführung des Verfahrens (β) setzt man die Aniline der Formel (VI) und die Azol-aldehyde der Formel (VII) im allgemeinen in äquimolaren Mengen ein. Es ist jedoch auch möglich, eine der Komponenten, vorzugsweise das Anilin der Formel (VI), in einem Überschuß einzusetzen. Das in der zweiten Stufe benötigte Reduktionsmittel wird zweckmäßigerweise in einem Überschuß eingesetzt. — Die Aufarbeitung und Isolierung der Reaktionsprodukte erfolgt jeweils nach üblichen Methoden.

Die außerdem für das erfindungsgemäße Verfahren (a) als Ausgangsstoffe zu verwendenden Verbindungen Chloressigsäurechlorid und -bromid bzw. -anhydrid sind durch die Formeln (IIIa) und (IIIb) definiert.

Das Chloressigsäurechlorid und -bromid bzw. -anhydrid der Formeln (IIIa) und (IIIb) sind allgemein bekannte Verbindungen der organischen Chemie.

Die bei der Durchführung des erfindungsgemäßen Verfahrens (b) als Ausgangsstoffe benötigten Chloracetanilide sind durch die Formel (IV) allgemein definiert. In dieser Formel stehen $\underline{R}^1$, $\underline{R}^2$ und $\underline{R}^3$ für diejenigen Reste, die bereits im Zusammenhang mit der Beschreibung der erfindungsgemäßen Stoffe der Formel (I) für diese Reste genannt wurden.

Die Chloracetanilide der Formel (IV) sind allgemein bekannt bzw. können sie in allgemein bekannter Art und Weise erhalten werden, indem man entsprechende Aniline mit Chloressigsäurechlorid oder -bromid bzw. -anhydrid der Formeln (IIIa) bzw. (IIIb) in Gegenwart eines inerten organischen Lösungsmittels, wie beispielsweise Toluol oder Dimethylformamid, gegebenenfalls in Gegenwart eines Säurebindemittels, wie z. B. Kaliumcarbonat oder Triethylamin, bei Temperaturen zwischen 0 und 100° C umsetzt (vgl. auch die Herstellungsbeispiele).

Die außerdem für das erfindungsgemäße Verfahren (b) als Ausgangsstoffe zu verwendenden Azolylalkyl-Derivate sind durch die Formel (V) allgemein definiert. In dieser Formel stehen $\underline{A}$, $\underline{X}^1$ und $\underline{X}^2$ für diejenigen Reste, die bereits im Zusammenhang mit der Beschreibung der erfindungsgemäßen Stoffe der Formel (I) genannt wurden. $\underline{Y}$ steht vorzugsweise für Chlor, Brom, den Mesylat- und Tosylat-Rest.

Die Azolyl-alkyl-Derivate der Formel (V) sind bekannt (vergleiche u. a. J. Chem. Soc. 1965, 7274 und 1978, 994; DE-OS 2 539 962 und C. A. 50, 3402 i); bzw. können sie in allgemein bekannter Art und Weise erhalten werden, indem man z. B. die entsprechenden Methyl-Derivate nach üblichen Methoden halogeniert.

Als Verdünnungsmittel kommen für die erfindungsgemäße Umsetzung gemäß Verfahrensvariante (a) vorzugsweise inerte organische Lösungsmittel in Frage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol, halogenierte Kohlenwasserstoffe, wie Methylenchlorid, Tetrachlorkohlenstoff, Chloroform oder Chlorbenzol; und Ester, wie Essigester.

Das erfindungsgemäße Verfahren (a) kann gegebenenfalls in Gegenwart von Säurebindern (Chlorwasserstoff-Akzeptoren) durchgeführt werden. Als solche können alle üblichen Säurebindemittel verwendet werden. Hierzu gehören vorzugsweise organische Basen, wie tertiäre Amine, beispielsweise Triethylamin, oder wie Pyridin ferner anorganische Basen, wie beispielsweise Alkalihydroxide und Alkalicarbonate.

Die Reaktionstemperaturen können bei der Durchführung des erfindungsgemäßen Verfahrens (a) in einem größeren Bereich variiert werden. Im allgemeinen arbeitet man zwischen 0 und 120° C, vorzugsweise zwischen 20° C und 100° C.

Bei der Durchführung des erfindungsgemäßen Verfahrens (a) setzt man vorzugsweise auf 1 Mol der Verbindung der Formel (II) 1 bis 1,5 Mol Chloracetylierungsmittel und gegebenenfalls 1 bis 1,5 Mol Säurebinder ein. Die Isolierung der Verbindungen der Formel (I) erfolgt in üblicher Weise.

Als Verdünnungsmittel kommen für die erfindungsgemäße Umsetzung gemäß Verfahren (b) alle inerten, mit Wasser nicht mischbaren, organischen Lösungsmittel in Frage.

Hierzu gehören vorzugsweise Ether, wie Diethylether; aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Die erfindungsgemäße Umsetzung gemäß Verfahren (b) wird in Gegenwart eines Säurebindemittels durchgeführt. Als solche können alle üblichen Säurebindemittel verwendet werden. Hierzu gehören vorzugsweise anorganische Basen, wie beispielsweise Alkylihydroxide und Alkalicarbonate.

Die Reaktionstemperaturen können bei der Durchführung des erfindungsgemäßen Verfahrens (b) in einem größeren Bereich variiert werden. Im allgemeinen arbeitet man zwischen −70 und +100° C, vorzugsweise zwischen −20° C und +80° C.

Bei der Durchführung des erfindungsgemäßen Verfahrens (b) setzt man vorzugsweise auf 1 Mol Chloracetanilid der Formel (IV) 1 bis 1,5 Mol Azolyl-alkyl-Derivate der Formel (V) ein. Die Isolierung der Verbindungen der Formel (I) erfolgt in üblicher Weise.

In einer bevorzugten Ausführungsform wird die erfindungsgemäße Umsetzung gemäß Verfahren (b) in einem Zweiphasensystem, wie beispielsweise wäßrige Natron- oder Kalilauge/Toluol oder

Methylenchlorid, gegebenenfalls unter Zusatz von 0,1 – 1 Mol eines Phasen-Transferkatalysators, wie beispielsweise Ammonium- oder Phosphoniumverbindungen, beispielsweise seien Benzyl-dodecyldimethyl-ammoniumchlorid (Zephirol) und Triethyl-benzyl-ammoniumchlorid genannt, durchgeführt (vgl. auch die Herstellungsbeispiele).

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Protulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuß-, Bananen-, Kaffee-, Tee-, Gummi-, Ölplam-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen insbesondere neben einer guten Wirkung gegen grasartige Unkräuter auch eine gute herbizide Wirkung bei breitblättrigen Unkräutern. Ein selektiver Einsatz der erfindungsgemäßen Wirkstoffe ist möglich, vorzugsweise in Mais, Erdnüssen, Rüben, Sojabohnen, Baumwolle, Reis und andere Getreidearten.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischen Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischungen mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,1 und 10 kg Wirkstoff pro ha, vorzugsweise zwischen 0,1 und 5 kg/ha.

In dem nachfolgenden Beispiel wird die nachstehend angegebene Verbindung als Vergleichssubstanz eingesetzt:

A = 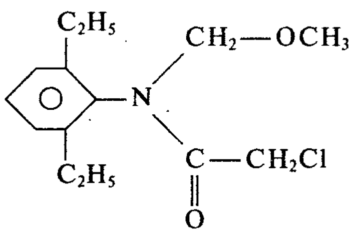

(2,6-Diethyl)-N-methoxymethyl-chloracetanilid

Beispiel A

Pre-emergence-Test

Lösungsmittel: 5 Gewichtsteile Aceton
Emulgator: 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesägt und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Wirkstoffe, Aufwandmengen und Resultate werden ermittelt.

Die erfindungsgemäßen Wirkstoffe (5) und (2) zeigen in diesem Test eine bessere selektive Wirksamkeit als die aus dem Stand der Technik bekannte Substanz (A).

## Herstellungsbeispiele

### Beispiel 1

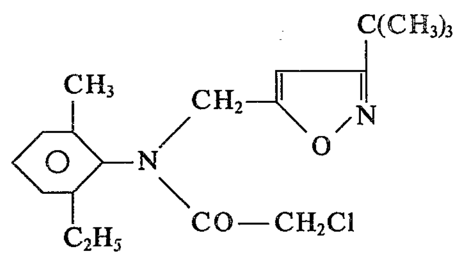

(Verfahren a)

13,6 g (0,05 Mol) 2-Ethyl-6-methyl-N-(3'-tert.-butyl-isoxazol-5'-yl-methyl)-anilin und 5,2 g (0,071 Mol) Pyridin werden in 50 ml Toluol vorgelegt und unter Rühren und Kühlung bei 0 – 5° C mit 6,2 g (0,055 Mol) Chloracetylchlorid versetzt. Man läßt zwei Stunden bei Raumtemperatur nachrühren und trennt den gebildeten Niederschlag ab. Das Filtrat wird mit Wasser gewaschen, über Natriumsulfat getrocknet und durch Abdestillieren des Lösungsmittels im Vakuum eingeengt. Der Rückstand wird aus Petrolether umkristallisiert. Man erhält 8,1 g (49% der Theorie) 2-Ethyl-6-methyl-N-(3'-tert.-butyl-isoxazol-5'-yl-methyl)-chloracetanilid vom Schmelzpunkt 78 – 79° C.

### Herstellung des Ausgangsproduktes

(II-1)

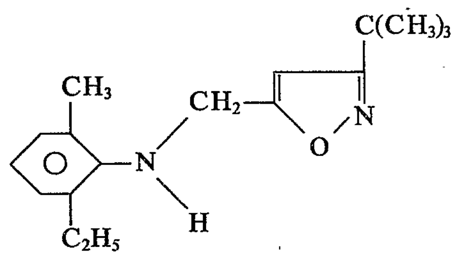

(Verfahren α)

65,1 g (0,48 Mol) 2-Ethyl-6-methylanilin, 33 g (0,19 Mol) 5-Chlormethyl-3-tert.-butyl-isoxazol und 26,2 g (0,19 Mol) gepulvertes Kaliumcarbonat werden in 48 ml Dimethylformamid 5 Stunden unter Rühren auf 100°C erhitzt. Danach wird die Reaktionsmischung auf 150 ml Wasser gegossen und mit Methylenchlorid extrahiert. Die organische Phase wird über Natriumsulfat getrocknet und durch Abdestillieren des Lösungsmittels im Vakuum eingeengt. Der Rückstand wird im Vakuum destilliert. Man erhält 35,2 g (68% der Theorie) 2-Ethyl-6-methyl-N-(3'-tert.-butyl-isoxazol-5'-yl-methyl)-anilin vom Siedepunkt 140 – 141° C/0,1 mm mit einer 95%igen Reinheit (Gaschromatographisch bestimmt) und einem Brechungsindex von $n_D^{20} = 1,5277$.

### Beispiel 2

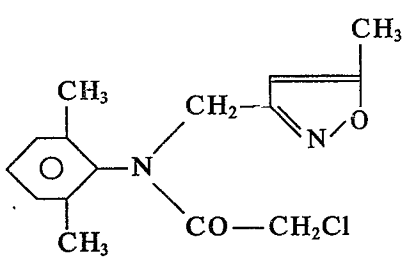

(Verfahren a)

6,48 g (0,03 Mol) 2,6-Dimethyl-N-(5'-methyl-isoxazol-3'-yl-methyl)-anilin und 2,4 g (0,033 Mol) Pyridin werden in 100 ml Tetrahydrofuran zum Sieden erhitzt und unter Rühren tropfenweise mit 3,8 g (0,033

Mol) Chloracetylchlorid versetzt. Nach 15 Minuten wird die Reaktionsmischung im Vakuum eingeengt und der Rückstand mit Wasser aufgenommen. Der entstehende kristalline Niederschlag wird abgesaugt und getrocknet. Man erhält 8,2 g (93% der Theorie) 2,6-Dimethyl-N-(5'-methyl-isoxazol-3'-yl-methyl)-chloracetanilid vom Schmelzpunkt 73–76° C.

Herstellung des Ausgangsproduktes

(II-2)

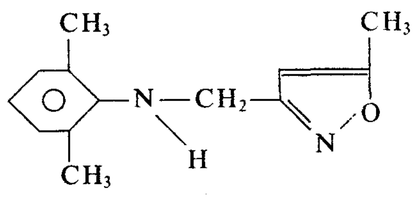

(Verfahren β)

13,7 g (0,064 Mol) 1-(5'-Methyl-isoxazol-3'-yl-methylenimino)-2,6-dimethylbenzol in 150 ml Methanol werden portionsweise mit 6 g (0,16 Mol) Natriumborhydrid versetzt bis dünnschichtchromatographisch kein Ausgangsprodukt mehr zu finden ist. Anschließend wird das Methanol abgezogen, der Rückstand zwischen Wasser und Methylenchlorid verteilt, die organische Phase abgetrennt, über Natriumsulfat getrocknet und im Vakuum eingeengt. Man erhält 11,6 g (84% der Theorie) 2,6-Dimethyl-N-(5'-methyl-isoxazol-3'-yl-methyl)-anilin vom Schmelzpunkt 49–51° C.

(VIII-1)

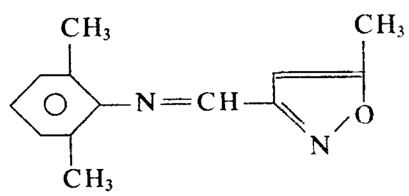

10,9 g (0,09 Mol) 2,6-Dimethylanilin und 10,6 g (0,09 Mol) 5-Methyl-isoxazol-3-carboxaldehyd werden nach Zusatz von 0,1 g Toluolsulfonsäure in 100 ml Toluol 2 Stunden am Wasserabscheider erhitzt. Anschließend wird das Reaktionsgemisch eingeengt und der Rückstand destilliert. Man erhält 15,1 g (78% der Theorie) 1-(5'-Methyl-isoxazol-3'-yl-methylenimino)-2,6-dimethylbenzol vom Siedepunkt 118–125° C/0,4 mm.

Beispiel 3

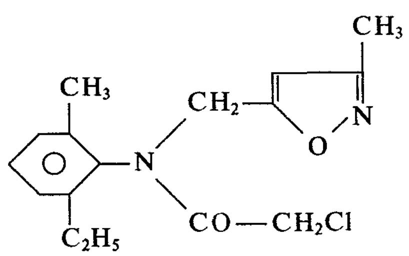

(Verfahren b)

2,4 g (0,011 Mol) 2-Ethyl-6-methyl-chloracetanilid werden in einem Zweiphasengemisch aus 20 ml Toluol und 10 ml 50%iger Natronlauge nach Zugabe von 0,1 g Triethyl-benzylammoniumchlorid gelöst und unter heftigem Rühren tropfenweise mit 2 g (0,011 Mol) 5-Brommethyl-3-methyl-isoxazol versetzt. Man läßt 3 Stunden bei Raumtemperatur nachrühren. Anschließend wird die Toluolphase abgetrennt, mehrmals mit Wasser gewaschen und über Natriumsulfat getrocknet. Nach Abdestillieren des Lösungsmittels im Vakuum erhält man 1,9 g (56% der Theorie) 2-Ethyl-6-methyl-N-(3'-methyl-isoxazol-5'-yl-methyl)-chloracetanilid vom Schmelzpunkt 78–79° C.

Herstellung des Ausgangsproduktes

(IV-1)

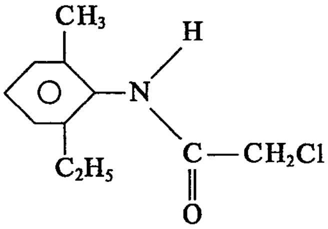

135,2 g (1 Mol) 2-Ethyl-6-methyl-anilin in 1000 ml Toluol werden mit 152 g (1,1 Mol) Kaliumcarbonat versetzt. Dazu werden unter Rühren 113 g (1 Mol) Chloressigsäurechlorid getropft. Nach Abklingen der exothermen Reaktion läßt man 2 Stunden unter Rückfluß nachrühren. Anschließend wird das Reaktionsgemisch filtriert und das Filtrat im Vakuum auf 500 ml eingeengt. Die dabei entstehenden Kristalle werden abgesaugt und mit Petrolether gewaschen. Man erhält 189,6 g (98% der Theorie) 2-Ethyl-6-methyl-chloracetanilid in Form weißer Kristalle vom Schmelzpunkt 120° C.

In entsprechender Weise werden diejenigen Verbindungen erhalten, die in der Tabelle 1 formelmäßig aufgeführt sind.

Tabelle 1

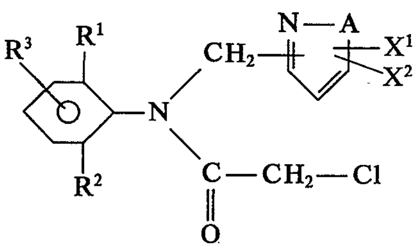

(I)

| Bsp. Nr. | $R^1$ | $R^2$ | $R^3$ | 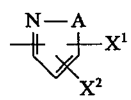 | Schmelzpunkt (°C) |
|---|---|---|---|---|---|
| 4 | $CH_3$ | $C_2H_5$ | H | 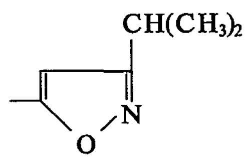 | 50 |
| 5 | $C_2H_5$ | $C_2H_5$ | H | 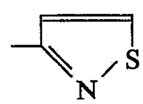 | 50–56 |
| 6 | $C_2H_5$ | $C_2H_5$ | H | 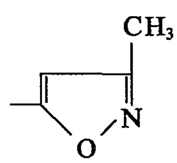 | 104 |
| 7 | $CH_3$ | $C_2H_5$ | H | 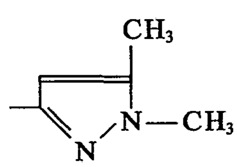 | viskoses Öl |
| 8 | $CH_3$ | $C_2H_5$ | H | 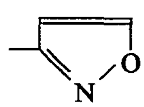 | 56–59 |

Fortsetzung

| Bsp. Nr. | $R^1$ | $R^2$ | $R^3$ | 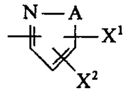 | Schmelzpunkt (°C) |
|---|---|---|---|---|---|
| 9 | $C_2H_5$ | $C_2H_5$ | H | 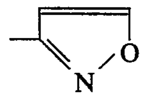 | viskoses Öl |
| 10 | $C_2H_5$ | $C_2H_5$ | H | 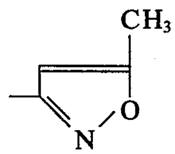 | 100–101 |
| 11 | $CH_3$ | $C_2H_5$ | H | 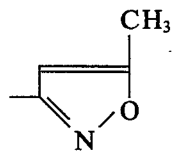 | 81–83 |
| 12 | $CH_3$ | $CH_3$ | H | 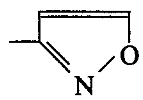 | 94–96 |
| 13 | $CH_3$ | $CH_3$ | H | 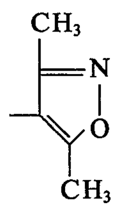 | 116–118 |
| 14 | $CH_3$ | $C_2H_5$ | H | 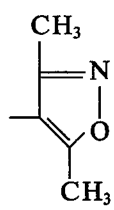 | 104–106 |
| 15 | $CH_3$ | $C_2H_5$ | H | 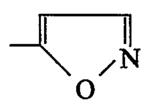 | 66–68 |
| 16 | $CH_3$ | H | 3-$CH_3$ | 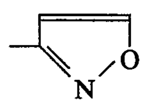 | 82–84 |
| 17 | $C(CH_3)_3$ | H | H | 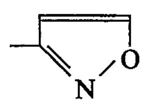 | 70 |

Nach einem oder mehreren der in der Anmeldung beschriebenen Verfahren und entsprechend den Beispielen 1 und 2 werden die in der nachstehenden Tabelle 2 formelmäßig aufgeführten Ausgangsprodukte der Formel (II) erhalten.

Tabelle 2

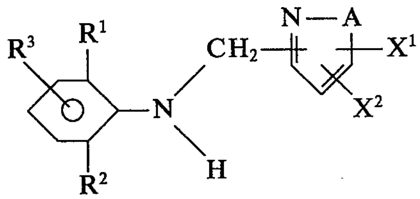

(II)

| Bsp. Nr. | $R^1$ | $R^2$ | $R^3$ | 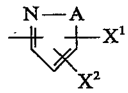 | Physikalische Konstante |
|---|---|---|---|---|---|
| (II-4) | $CH_3$ | $C_2H_5$ | H | 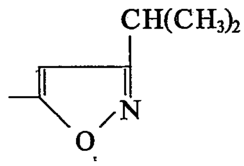 | Kp: 140–42°C/ 0,05 mm |
| (II-5) | $C_2H_5$ | $C_2H_5$ | H | 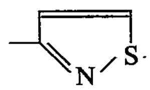 | Kp: 140°C/ 0,05 mm $n_D^{20}$ = 1,5800 |
| (II-6) | $C_2H_5$ | $C_2H_5$ | H | 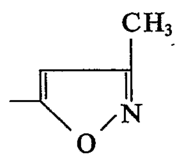 | Kp: 123–30°C/ 0,05 mm $n_D^{20}$ = 1,5442 |
| (II-7) | $CH_3$ | $C_2H_5$ | H | 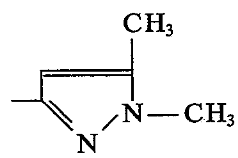 | zähes Öl |
| (II-8) | $CH_3$ | $C_2H_5$ | H | 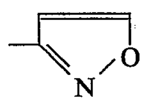 | Kp: 118–20°C/ 0,1 mm |
| (II-9) | $C_2H_5$ | $C_2H_5$ | H | 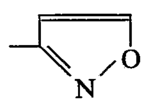 | Kp: 120–25°C/ 0,1 mm |
| (II-10) | $C_2H_5$ | $C_2H_5$ | H | 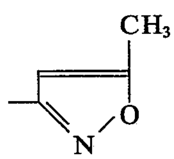 | Kp: 125–29°C/ 0,1 mm |
| (II-11) | $CH_3$ | $C_2H_5$ | H | 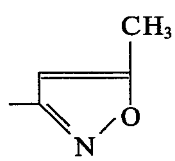 | Kp: 130–50°C/ 0,1 mm |

Fortsetzung

| Bsp. Nr. | $R^1$ | $R^2$ | $R^3$ | 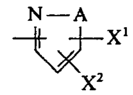 | Physikalische Konstante |
|---|---|---|---|---|---|
| (II-12) | $CH_3$ | $CH_3$ | H | 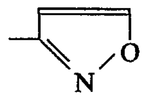 | Fp: 73–76°C |
| (II-13) | $CH_3$ | $CH_3$ | H | 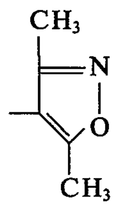 | Fp: 78–81°C |
| (II-14) | $CH_3$ | $C_2H_5$ | H | 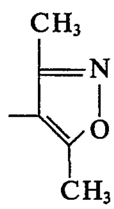 | Kp: 156–157°C/ 0,2 mm |
| (II-15) | $CH_3$ | $C_2H_5$ | H | 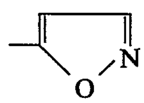 | Kp: 120°C/ 0,1 mm |
| (II-16) | $CH_3$ | H | 3-$CH_3$ | 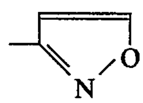 | Kp: 160°C/ 0,2 mm |
| (II-17) | $C(CH_3)_3$ | H | H | 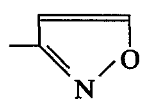 | Kp: 152–156°C/ 0,2 mm |

Nach bekannten Verfahren und entsprechend Beispiel 3 werden die in der nachstehenden Tabelle 3 formelmäßig aufgeführten Ausgangsprodukte der Formel (IV) erhalten.

Tabelle 3

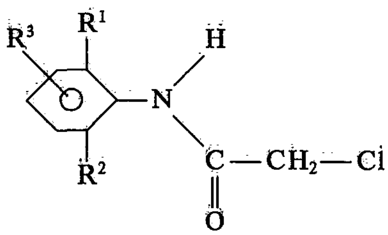

(IV)

| Beispiel Nr. | $R^1$ | $R^2$ | $R^3$ | Schmelzpunkt (°C) |
|---|---|---|---|---|
| (IV-2) | $CH_3$ | $CH_3$ | H | 148 |
| (IV-3) | $C_2H_5$ | $C_2H_5$ | H | 133 |
| (IV-4) | i-$C_3H_7$ | H | H | 79 |
| (IV-5) | tert.-$C_4H_9$ | H | H | 96 |
| (IV-6) | $C_2H_5$ | H | H | 103 |
| (IV-7) | $CH_3$ | H | H | 109 |
| (IV-8) | $CH_3$ | H | 3-$CH_3$ | 135 |
| (IV-9) | $CH_3$ | H | 5-$CH_3$ | 154 |
| (IV-10) | $CH_3$ | $CH_3$ | 4-$CH_3$ | 177 |
| (IV-11) | $C_2H_5$ | $CH_3$ | 4-$CH_3$ | 134 |
| (IV-12) | sek.-$C_4H_9$ | H | H | Öl |
| (IV-13) | H | H | H | 132 |

**Patentansprüche**

1. N-(1,2-Azolyl)-alkyl-chloracetanilide der Formel

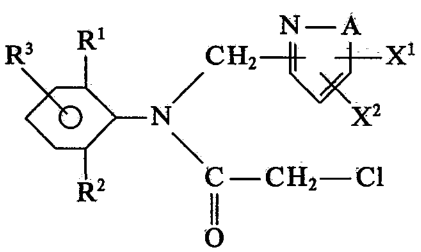

(I)

in welcher

A für Sauerstoff, Schwefel oder die Gruppierung >NR steht, wobei
R für Wasserstoff oder Alkyl mit 1 bis 4 Kohlenstoffatomen steht,
$R^1$ für Alkyl mit 1 bis 4 Kohlenstoffatomen steht,
$R^2$ für Wasserstoff oder Alkyl mit 1 bis 4 Kohlenstoffatomen steht,
$R^3$ für Wasserstoff oder Alkyl mit 1 bis 4 Kohlenstoffatomen steht,
$X^1$ für Wasserstoff oder Alkyl mit 1 bis 4 Kohlenstoffatomen steht,
$X^2$ für Wasserstoff oder Alkyl mit 1 bis 4 Kohlenstoffatomen steht und der Azolyl-Rest über ein Kohlenstoffatom gebunden ist.

2. 2-Ethyl-6-methyl-N-(3'-methyl-isoxazol-5'-yl-methyl)-chloracetanilid der Formel

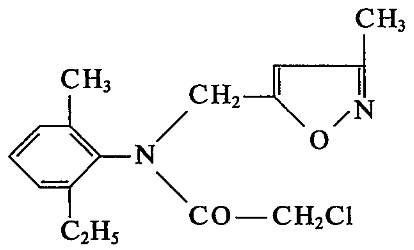

3. 2,6-Diethyl-N-(thiazol-3-yl-methyl)-chloracetanilid der Formel

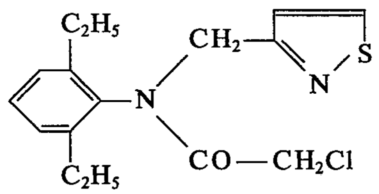

4. Verfahren zur Herstellung von N-(1,2-Azolyl)-alkylchloracetaniliden der Formel

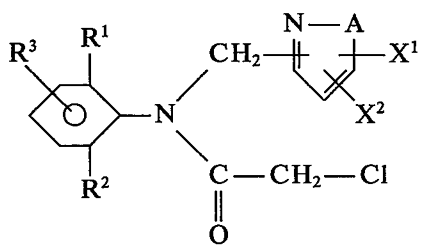

(I)

in welcher

A für Sauerstoff, Schwefel oder die Gruppierung >NR steht, wobei
R für Wasserstoff oder Alkyl mit 1 bis 4 Kohlenstoffatomen steht,
$R^1$ für Alkyl mit 1 bis 4 Kohlenstoffatomen steht,
$R^2$ für Wasserstoff oder Alkyl mit 1 bis 4 Kohlenstoffatomen steht,
$R^3$ für Wasserstoff oder Alkyl mit 1 bis 4 Kohlenstoffatomen steht,
$X^1$ für Wasserstoff oder Alkyl mit 1 bis 4 Kohlenstoffatomen steht,
$X^2$ für Wasserstoff oder Alkyl mit 1 bis 4 Kohlenstoffatomen steht und der Azolyl-Rest über ein Kohlenstoffatom gebunden ist,

dadurch gekennzeichnet, daß man

a) N-(1,2-Azolyl)-alkyl-aniline der Formel

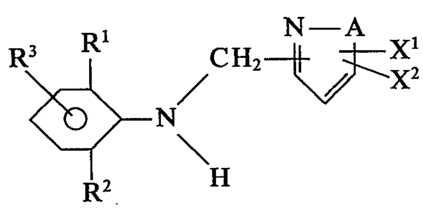

(II)

in welcher
A, $R^1$, $R^2$, $R^3$, $X^1$ und $X^2$ die oben angegebene Bedeutung haben, und der Azolyl-Rest über ein Kohlenstoffatom gebunden ist,
mit Chloressigsäurechlorid oder -bromid bzw. -anhydrid der Formeln

$$Cl-CH_2-CO-Cl(Br) \qquad \text{(IIIa)}$$

bzw.

$$(Cl-CH_2-CO)_2O \qquad \text{(IIIb)}$$

in Gegenwart eines Verdünnungsmittels und gegebenenfalls in Gegenwart eines Säurebindemittels umsetzt, oder

b) Chloracetanilide der Formel

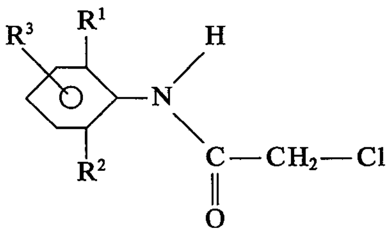

(IV)

in welcher
$R^1$, $R^2$ und $R^3$ die oben angegebene Bedeutung haben,
mit Azolyl-alkyl-Derivaten der Formel

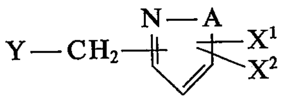

(V)

in welcher
A, $X^1$ und $X^2$ die oben angegebene Bedeutung haben,
Y für Halogen, den Mesylat- oder Tosylat-Rest steht,
und der Azolyl-Rest über ein Kohlenstoffatom gebunden ist,
in Gegenwart eines Säurebinders und gegebenenfalls in Gegenwart eines organischen Lösungsmittels umsetzt.

5. Herbizide Mittel, gekennzeichnet durch einen Gehalt an mindestens einem N-(1,2-Azolyl)-alkyl-chloracetanilid der Formel (I).

6. Verfahren zur Herstellung von herbiziden Mitteln, dadurch gekennzeichnet, daß man N-(1,2-Azolyl)-alkyl-chloracetanilide der Formel (I) mit Streckmitteln und/oder oberflächenaktiven Mitteln vermischt.

7. Verwendung von N-(1,2-Azolyl)-alkyl-chloracetaniliden der Formel (I) zur Bekämpfung von Unkräutern.

**Claims**

1. N-(1,2-Azolyl)-alkyl-chloracetanilides of the formula

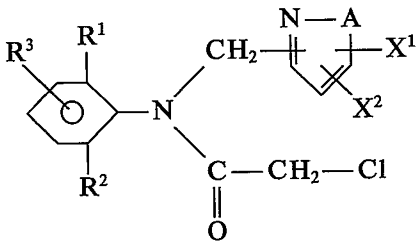

(I)

in which

A represents oxygen, sulphur or the grouping >NR,
R representing hydrogen or alkyl with 1 to 4 carbon atoms,
$R^1$ represents alkyl with 1 to 4 carbon atoms,
$R^2$ represents hydrogen or alkyl with 1 to 4 carbon atoms,
$R^3$ represents hydrogen or alkyl with 1 to 4 carbon atoms,
$X^1$ represents hydrogen or alkyl with 1 to 4 carbon atoms,
$X^2$ represents hydrogen or alkyl with 1 to 4 carbon atoms and the azolyl radical is bonded via a carbon atom.

2. 2-Ethyl-6-methyl-N-(3′-methyl-isoxazol-5′-yl-methyl)-chloroacetanilide of the formula

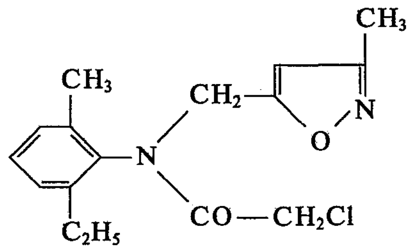

3. 2,6-Diethyl-N-(thiazol-3-yl-methyl)-chloroacetanilide of the formula

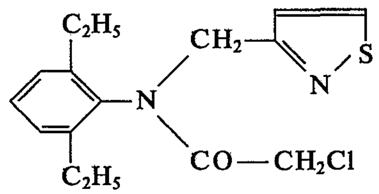

4. Process for the preparation of N-(1,2-azolyl)-alkylchloroacetanilides of the formula

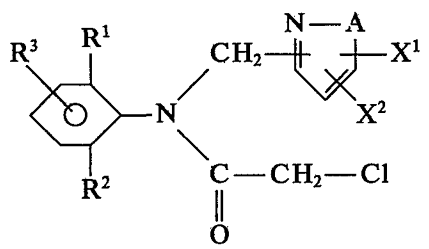

(I)

in which

A represents oxygen, sulphur or the grouping >NR,
R representing hydrogen or alkyl with 1 to 4 carbon atoms,
$R^1$ represents alkyl with 1 to 4 carbon atoms,
$R^2$ represents hydrogen or alkyl with 1 to 4 carbon atoms,
$R^3$ represents hydrogen or alkyl with 1 to 4 carbon atoms,
$X^1$ represents hydrogen or alkyl with 1 to 4 carbon atoms,
$X^2$ represents hydrogen or alkyl with 1 to 4 carbon atoms and the azolyl radical is bonded via a carbon atom,

characterised in that

a) N-(1,2-azolyl)-alkyl-anilines of the formula

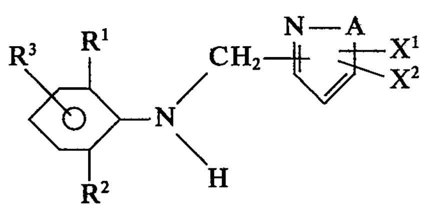

(II)

in which
A, $R^1$, $R^2$, $R^3$, $X^1$ and $X^2$ have the meaning indicated above, and the azolyl radical is bonded via a carbon atom,
are reacted with chloroacetic acid chloride or bromide or anhydride of the formulae

$$Cl—CH_2—CO—Cl(Br) \qquad (IIIa)$$

or

$$(Cl-CH_2-CO)_2O \qquad \text{(IIIb)}$$

in the presence of a diluent and if appropriate in the presence of an acid-binding agent, or

b) chloroacetanilides of the formula

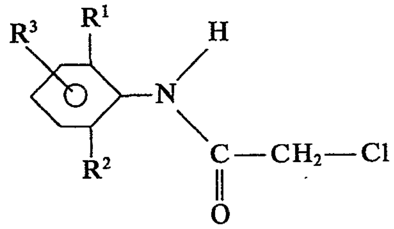

(IV)

in which
$R^1$, $R^2$ and $R^3$ have the meaning indicated above are reacted with azolyl-alkyl derivates of the formula

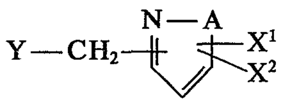

(V)

in which
A, $X^1$ and $X^2$ have the meaning indicated above,
Y represents halogen or the mesylate or tosylate radical,
and the azolyl radical is bonded via a carbon atom,
in the presence of an acid-binding agent and if appropriate in the presence of an organic solvent.

5. Herbicidal agents, characterised in that they contain at least one N-(1,2-azolyl)-alkyl-chloroacetanilide of the formula (I).

6. Process for the preparation of herbicidal agents, characterised in that N-(1,2-azolyl)-alkyl-chloroacetanilides of the formula (I) are mixed with extenders and/or surfaceactive agents.

7. Use of N-(1,2-azolyl)-alkyl-chloroacetanilides of the formula (I) for combating weeds.

**Revendications**

1. N-(1,2-azolyl)alkyl-chloracétanilides de formule:

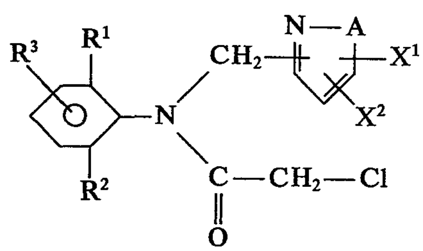

(I)

dans laquelle:

A est l'oxygène, le soufre ou le groupement >NR dans lequel
R est l'hydrogène ou un reste alkyle ayant 1 à 4 atomes de carbone,
$R^1$ est un reste alkyle ayant 1 à 4 atomes de carbone,
$R^2$ est l'hydrogène ou un reste alkyle ayant 1 à 4 atomes de carbone,
$R^3$ est l'hydrogène ou un reste alkyle ayant 1 à 4 atomes de carbone,
$X^1$ est l'hydrogène ou un reste alkyle ayant 1 à 4 atomes de carbone,
$X^2$ est l'hydrogène ou un reste alkyle ayant 1 à 4 atomes de carbone et le reste azolyle est lié par un atome de carbone.

2. Le 2-éthyl-6-méthyl-N-(3'-méthylisoxazol-5'-yl-méthyl)-chloracétanilide de formule:

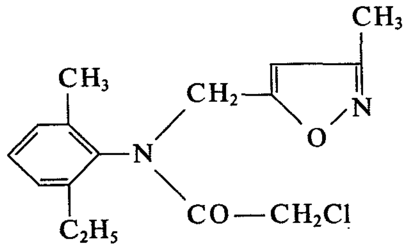

3. Le 2,6-diéthyl-N-(thiazolyl-3-ylméthyl)-chloracétanilide de formule:

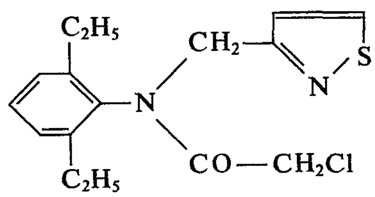

4. Procédé de production de N-(1,2-azolyl)-alkyl-chloracétanilides de formule:

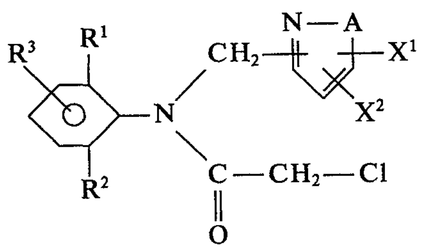

(I)

dans laquelle:

A est l'oxygène, le soufre ou le groupement > NR, dans lequel
R est l'hydrogène ou un reste alkyle ayant 1 à 4 atomes de carbone,
$R^1$ est un reste alkyle ayant 1 à 4 atomes de carbone,
$R^2$ est l'hydrogène ou un reste alkyle 1 à 4 atomes de carbone,
$R^3$ est l'hydrogène ou un reste alkyle ayant 1 à 4 atomes de carbone,
$X^1$ est l'hydrogène ou un reste alkyle ayant 1 à 4 atomes de carbone,
$X^2$ est l'hydrogène ou un reste alkyle ayant 1 à 4 atomes de carbone et le reste azolyle est lié par un atome de carbone,

caractérisé en ce que

a) on fait réagir des N-(1,2-azolyl)alkyl-anilines de formule:

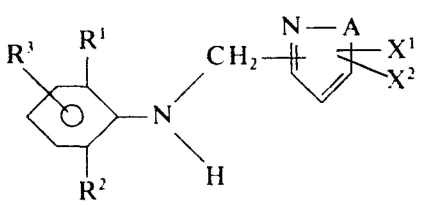

(II)

dans laquelle:
A, $R^1$, $R^2$, $R^3$, $X^1$ et $X^2$ ont la définition indiquée ci-dessus, et le reste azolyle est lié par un atome de carbone,
avec le chlorure ou bromure ou l'anhydride d'acide chloracétique de formules:

$$Cl—CH_2—CO—Cl(Br) \qquad (IIIa)$$

ou

$$(Cl-CH_2-CO)_2O \qquad \text{(IIIb)}$$

en présence d'un diluant et, le cas échéant, en présence d'un accepteur d'acide, ou

b) on fait réagir des chloracétanilides de formule:

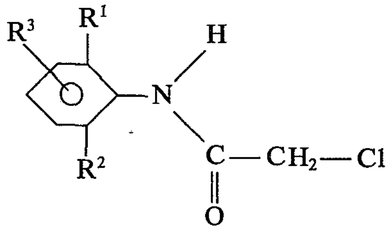

(IV)

dans laquelle:
$R^1$, $R^2$ et $R^3$ ont la définition indiquée ci-dessus, avec des dérivés azolyl-alkyliques de formule:

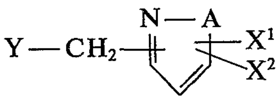

(V)

dans laquelle:
A, $X^1$ et $X^2$ ont la définition indiquée ci-dessus,
Y désigne un halogène, le reste mésylate ou le reste tosylate,
et le reste azolyle est lié par un atome de carbone, en présence d'un accepteur d'acide et, le cas échéant, en présence d'un solvant organique.

5. Compositions herbicides, caractérisées par une teneur en au moins un N-(1,2-azolyl)alkyl-chloracétanilide de formule (I).

6. Procédé de production de compositions herbicides, caractérisé en ce qu'on mélange des N-(1,2-azolyl)-alkyl-chloracétanilides de formule (I) avec des diluants et/ou des agents tensio-actifs.

7. Utilisation de N-(1,2-azolyl)alkyl-chloracétanilides de formule (I) pour lutter contre des mauvaises herbes.